# EUROPÄISCHE PATENTANMELDUNG

(11) **EP 2 016 978 A2**
(43) Veröffentlichungstag der Anmeldung: **21.01.2009**
(21) Anmeldenummer: 08104470.3
(22) Anmeldetag: 19.06.2008
(51) Int. Cl.: A61N 5/10

(54) **Partikelstrahlapplikationsvorrichtung, Bestrahlungsvorrichtung sowie Verfahren zur Führung eines Partikelstrahls**

(30) Priorität: 20.07.2007 DE 102007033895; 20.07.2007 US 961461 P
(71) Anmelder: SIEMENS AKTIENGESELLSCHAFT, 80333 München (DE)
(72) Erfinder: Hansmann, Thomas, 69117 Heidelberg (DE); Rietzel, Eike, 64289 Darmstadt (DE)

(57) **Zusammenfassung**

Die Erfindung betrifft eine Partikelstrahlapplikationsvorrichtung zum Formen und Führen eines Partikelstrahls, umfasst einen ersten Kollimator zum Formen eines Querschnittsprofils eines in den Kollimator eintretenden Partikelstrahls, wobei der erste Kollimator eine Öffnung aufweist, die einem zu bestrahlenden Zielvolumen angepasst ist, und ein Magnetsystem zur Umlenkung des Partikelstrahls, welches im Strahlverlauf des Partikelstrahls hinter dem ersten Kollimator angeordnet ist, wobei mit dem Magnetsystem ein Magnetfeld erzeugbar ist, mit dem der Partikelstrahl spektral auffächerbar ist. Weiterhin betrifft die Erfindung eine Bestrahlungsvorrichtung mit einer derartigen Partikelstrahlapplikationsvorrichtung sowie ein Verfahren zur Führung eines Partikelstrahls, bei dem ein Partikelstrahl durch einen Kollimator an ein Zielvolumen angepasst wird und anschließend durch ein Magnetsystem gelenkt wird, wodurch der Partikelstrahl von Streustrahlung gereinigt wird.

## Beschreibung

Die Erfindung betrifft eine Partikelstrahlapplikationsvorrichtung, wie sie insbesondere im Rahmen der Partikeltherapie zur Bestrahlung von Gewebe mit einem Partikelstrahl eingesetzt wird. Weiterhin betrifft die Erfindung eine Bestrahlungsvorrichtung mit einer derartigen Partikelstrahlapplikationsvorrichtung. Weiterhin betrifft die Erfindung ein Verfahren zur Führung eines Partikelstrahls, wie es insbesondere im Rahmen der Partikeltherapie eingesetzt wird.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, können jedoch auch in nicht-therapeutischen Gebieten Anwendung finden kann. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden üblicherweise geladene Partikel auf hohe Energien beschleunigt, zu einem Partikelstrahl geformt und auf ein zu bestrahlendes Objekt gerichtet. Der Partikelstrahl dringt in das Objekt ein und gibt dort an einem definierten Ort seine Energie ab, was zu einer Zerstörung des sich dort befindlichen Gewebes führt. Als Partikel kommen üblicherweise Protonen, Kohlenstoffionen aber auch Pionen, Heliumionen oder andere Ionensorten zum Einsatz.

Nachdem Partikel beschleunigt sind und ein Partikelstrahl geformt ist, besteht die Notwendigkeit, den Partikelstrahl möglichst zielgenau auf ein zu bestrahlendes Zielvolumen zu richten, damit die Energie des Partikelstrahls möglichst genau an einem vorgegebenen Zielort deponiert wird.

Ein bekanntes Verfahren ist dabei, den Partikelstrahl mit einer Streuvorrichtung kegelförmig aufzuweiten und anschließend auf das zu bestrahlende Zielvolumen zu richten, auch bekannt unter dem Namen "Scattering-Verfahren". Typischerweise umfasst eine Streuvorrichtung zwei Streuschichten. Die Streuvorrichtung und der Patient liegen hierbei auf einer Linie. Der Partikelstrahl wird durch Blenden bzw. Kollimatoren, die oftmals patientenspezifisch gefertigt und angepasst werden, in seiner Ausdehnung begrenzt.

Die Eindringtiefe des Partikelstrahls in ein zu bestrahlendes Zielvolumen hängt von der Energie ab, die der Partikelstrahl aufweist. Um den Partikelstrahl an ein z.B. in unterschiedlichen Tiefen gelegenes Zielvolumen im Zielkörper anzupassen, um also eine so genannte Tiefenabdeckung korrekt zu erreichen, werden in den Strahlengang des Partikelstrahls Elemente eingebracht, die die Energie des Partikelstrahls insbesondere ortsspezifisch abschwächen, z.B. durch sogenannte Kompensatoren. Außerdem ist zuvor oftmals eine generelle Aufweitung des Partikelstrahls in der Tiefe notwendig, um eine ausgedehnte Tiefenabdeckung zu erzielen. Zu derartigen Elementen gehören beispielsweise so genannte Modulator-Wheels oder Ridge-Filter.

An allen Elementen im Strahlengang des Partikelstrahls, an denen eine Wechselwirkung zwischen Materie mit dem Partikelstrahl stattfindet, entsteht Sekundärstrahlung. Diese Sekundärstrahlung, die beispielsweise Neutronenstrahlung umfassen kann, kann zu einer Energiedeposition der Partikel der Sekundärstrahlung im Zielvolumen an einem ungewollten Ort führen. Hierdurch wird die Qualität eines Bestrahlungsvorgangs eingeschränkt.

Es ist daher die Aufgabe der Erfindung, eine Partikelstrahlapplikationsvorrichtung anzugeben, mit der ein Partikelstrahl auf ein zu bestrahlendes Zielvolumen gerichtet werden kann, wobei das Zielvolumen mit nur geringer Sekundärstrahlung belastet wird. Weiterhin ist es die Aufgabe der Erfindung, eine Bestrahlungsvorrichtung anzugeben, mit der eine Bestrahlung eines Zielvolumens mit nur geringer Belastung durch Sekundärstrahlung durchgeführt werden kann. Weiterhin ist es die Aufgabe der Erfindung ein Verfahren zur Führung eines Partikelstrahls anzugeben, so dass der Partikelstrahl nur eine geringe Kontamination durch Sekundärstrahlung aufweist. Die Aufgabe wird erfindungsgemäß gelöst durch eine Partikelstrahlapplikationsvorrichtung nach Anspruch 1, durch eine Bestrahlungsvorrichtung nach Anspruch 11 sowie durch ein Verfahren nach Anspruch 12.

Die erfindungsgemäße Partikelstrahlapplikationsvorrichtung zum Formen und Führen eines Partikelstrahls, so dass der Partikelstrahl insbesondere auf ein zu bestrahlendes Zielvolumen richtbar ist, umfasst:
- einen ersten Kollimator zum Formen eines Querschnittsprofils eines in den Kollimator eintretenden Partikelstrahls, wobei der erste Kollimator eine Öffnung aufweist, die insbesondere einem zu bestrahlenden Zielvolumen angepasst ist,
- ein Magnetsystem zur Umlenkung des Partikelstrahls, welches im Strahlverlauf des Partikelstrahls hinter dem ersten Kollimator angeordnet ist, wobei mit dem Magnetsystem ein Magnetfeld erzeugbar ist, mit dem der Partikelstrahl spektral auffächerbar ist.

Der Erfindung liegt folglich die Idee zu Grunde, dass ein Partikelstrahl, der durch einen Kollimator hindurch tritt, durch Streustrahlung verunreinigt wird. Um den von Streustrahlung verunreinigten Partikelstrahl wieder zu reinigen, wird der Partikelstrahl durch ein Magnetfeld spektral aufgefächert. Dies bedeutet, dass die Komponenten des Partikelstrahls nach ihrem Masse-zu-Ladungs-Verhältnis und/oder nach ihrem Impuls unterschiedlich im Magnetfeld abgelenkt werden. Hierdurch kann die Streustrahlung, die sich im Partikelstrahl befindet, von den Partikeln, die zur Bestrahlung eingesetzt werden, räumlich getrennt werden. Dies beruht darauf, dass die Streustrahlung ein anderes Masse-zu-Ladungs-Verhältnis und/oder eine andere Energie bzw. Impuls als die Partikel, mit denen eine Bestrahlung durchgeführt wird, die ein gewünschtes Masse-zu-Ladungs-Verhältnis und eine gewünschte Energie bzw. Impuls aufweisen. Zur Streustrahlung gehören z.B. auch Elektronen oder Fragmente, die aus dem Material des Kollimators entstehen. Hierzu gehören aber auch ungeladene Teilchen, wie beispielsweise Photonen oder Neutronen, die im Magnetfeld dann keine Umlenkung erfahren.

Der Kollimator kann dabei jedoch auch als einfache Blende mit einer rechteckigen Öffnung ausgebildet sein, z.B. mit einer einfachen Schlitzblende.

Nach diesem Prinzip wird eine vorteilhafte Ausführungsvariante der Partikelstrahlapplikationsvorrichtung ermöglicht. Der erste Kollimator zum Formen des Querschnittsprofils des in den ersten Kollimator eintretenden Partikelstrahls kann dabei derart ausgebildet sein, dass der Kollimator die Energie von den Partikeln des Partikelstrahls, die nicht durch die Eröffnung des Kollimators treten, lediglich abschwächt.

In dieser Ausführungsvariante unterscheidet sich der Kollimator deutlich von bisher in der Partikeltherapie eingesetzten Kollimatoren, die diejenigen Partikel des Partikelstrahls, die nicht durch die Öffnung des Kollimators treten, vollständig aus dem Partikelstrahl entfernen. Bei dem nun eingesetzten Kollimator werden die Partikel, die nicht durch die Öffnung des Kollimators treten, lediglich in ihrer Energie abgeschwächt. Die räumliche Separation dieser Partikel von den Partikeln, die durch die Öffnung des Kollimators hindurch geführt worden sind, erfolgt anschließend durch das Magnetfeld, das den Partikelstrahl umlenkt. Der Vorteil dieser Ausführungsvariante ist, dass der Kollimator deutlich schlanker und damit kostengünstiger hergestellt werden kann, da er lediglich die Energie von Partikeln abschwächen muss und diese Partikel nicht vollständig - beispielsweise durch Absorption - aus dem Partikelstrahl entfernen muss.

Dennoch ist auch ein Einsatz eines herkömmlichen Kollimators, mit dem Partikel, die nicht durch die Öffnung des Kollimators treten aus dem Partikelstrahl entfernt werden, möglich.

In einer Ausführungsvariante beträgt die durch das Magnetsystem erzeugte Umlenkung des Partikelstrahls mehr als 5°, insbesondere mehr als 10°. Hierdurch ergibt sich eine effektive räumliche Separation der Streustrahlung von gewünschten Komponenten des Partikelstrahls. Das Magnetsystem, das das Magnetfeld erzeugt, kann dabei zumindest einen Dipolmagneten umfassen.

Der erste Kollimator kann dabei ein Kollimator sein, der individuell für das zu bestrahlende Zielvolumen gefertigt wird. Hierdurch ist eine genaue Anpassung des Kollimators an das zu bestrahlende Zielvolumen gewährleistet. Als Kollimator kann aber auch ein Multi-Leaf-Kollimator eingesetzt werden, der eine flexible Anpassung an das zu bestrahlende Zielvolumen erlaubt. Als weitere Möglichkeit kann als Kollimator ein aus vorgefertigten Elementen zusammensetzbarer Kollimator eingesetzt werden. Auch hierdurch lässt sich eine Anpassung des Kollimators und insbesondere seiner Öffnung an das zu bestrahlende Zielvolumen erreichen, ohne dass der Kollimator stets neu gefertigt wird.

Insbesondere weist die Partikelstrahlapplikationsvorrichtung weiterhin eine Strahlaufweitungsvorrichtung auf, die im Strahlverlauf vor dem ersten Kollimator angeordnet ist. Die Strahlaufweitungsvorrichtung kann dabei eine oder mehrere Streuschichten umfassen. Die durch die Strahlaufweitungsvorrichtung entstehende Streustrahlung wird durch die nachfolgenden Komponenten effektiv aus dem Partikelstrahl entfernt.

In einer vorteilhaften Ausführungsform weist die Partikelstrahlapplikationsvorrichtung zusätzlich einen zweiten Kollimator auf, mit dem das Querschnittsprofil des aus dem Magnetsystem austretenden Partikelstrahls begrenzbar ist. Der zweite Kollimator ist im Strahlverlauf hinter dem Magnetsystem angeordnet.

Durch den zweiten Kollimator wird das Querschnittsprofil des Partikelstrahls erneut geformt und kann dadurch verbessert werden. Insbesondere, wenn ein zweiter Kollimator verwendet wird, muss der erste Kollimator weniger genau ausgeführt sein und das Querschnittsprofil des Partikelstrahls weniger genau formen, da etwaige Ungenauigkeiten durch den zweiten Kollimator ausgeglichen werden können. Umgekehrt kann der zweite Kollimator in seiner strahlbegrenzenden Eigenschaft weniger genau ausgeführt sein, wenn das Querschnittsprofil des Partikelstrahls durch den ersten Kollimator bereits genau geformt ist.

Insgesamt lässt sich durch den zweiten Kollimator der Partikelstrahl nochmals genauer an das zu bestrahlende Zielvolumen anpassen. Insbesondere kann eine geometrische Aufweitung des Partikelstrahls, die nach dem Strahlensatz nach Zurücklegen einer Strecke auftritt, erneut ausgeglichen werden.

Ebenso wie der erste Kollimator kann der zweite Kollimator ein individuell für das zu bestrahlende Zielvolumen gefertigter Kollimator, ein aus vorgefertigten Elementen zusammensetzbarer Kollimator oder ein Multi-Leaf-Kollimator sein.

Die erfindungsgemäße Bestrahlungsvorrichtung umfasst zumindest eine Partikelstrahlapplikationsvorrichtung nach einem der Ansprüche 1 bis 10, zumindest eine Quelle zur Erzeugung von Partikeln, und zumindest eine vor der Partikelstrahlapplikationsvorrichtung angeordnete Beschleunigungsvorrichtung zur Beschleunigung der Partikel und zur Erzeugung des Partikelstrahls aus den beschleunigten Partikeln.

Das erfindungsgemäße Verfahren zur Führung eines Partikelstrahls umfasst folgende Verfahrensschritte:
- Formen eines Querschnittsprofils eines Partikelstrahls insbesondere derart, dass der Partikelstrahl an ein zu bestrahlendes Zielvolumen angepasst ist, und
- Führen des geformten Partikelstrahls durch ein Magnetfeld, wodurch der Partikelstrahl umgelenkt und spektral aufgefächert wird.

Dies bedeutet, dass Komponenten bzw. Teilchen des Partikelstrahls mit einem ungewünschten Masse-zu-Ladungs-Verhältnis bzw. einer ungewünschten Energie von Partikeln, die ein Zielvolumen bestrahlen sollen, räumlich getrennt werden.

Das Formen des Querschnittsprofils des Partikelstrahls kann durch einen Kollimator erfolgen, durch dessen Öffnung der Partikelstrahl hindurch tritt. Der Kollimator kann dabei so beschaffen sein, dass diejenigen Partikel, die nicht durch die Öffnung des Kollimators treten in ihrer Energie lediglich abgeschwächt werden anstatt vollständig aus dem Partikelstrahl entfernt zu werden. Vor dem Formen des Querschnittsprofils wird der Partikelstrahl zweckmäßigerweise aufgeweitet.

Nach dem spektralen Auffächern des Partikelstrahls durch das Magnetfeld kann das Querschnittsprofil des Partikelstrahls erneut geformt werden. Diesmal wird der Partikelstrahl in seinem Querschnittsprofil mit einem Kollimator derart geformt, dass Partikel, die nicht durch die Öffnung des Kollimators treten, aus dem Partikelstrahl entfernt werden, indem sie beispielsweise durch den Kollimator absorbiert werden.

Die Energie der Partikel des Partikelstrahls kann insbesondere vor Umlenkung des Partikelstrahls durch eine Tiefenmodulationsvorrichtung eingestellt werden. Es sind aber auch andere Anordnungen zur Tiefenmodulationsvorrichtung möglich, wie z.B. nach der Umlenkung.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: einen schematischen Überblick über eine Partikelthera- pieanlage,
- Fig. 2: einen schematischen Aufbau einer Ausführungsform einer Partikelstrahlapplikationsvorrichtung,
- Fig. 3: und Fig. 4 jeweils einen schematischen Aufbau einer weiteren Ausführungsform einer Partikelstrahlapplika- tionsvorrichtung, und
- Fig. 5: einen schematischen Überblick über die Verfahrens- schritte.

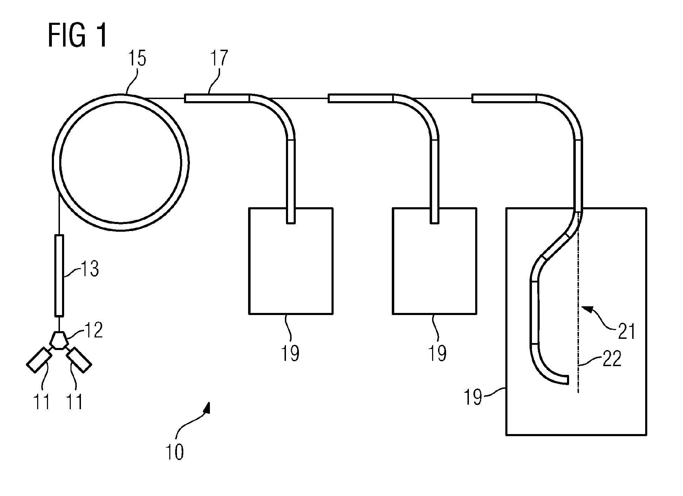

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen; beispielsweise muss, je nach Beschleunigung der Partikel, eine Bestrahlungsvorrichtung nicht als Partikeltherapieanlage angeordnet sein.

Die nachfolgend beschriebenen Ausführungsbeispiele sind sowohl in Zusammenhang mit der anhand der Figur 1 dargestellten Partikeltherapieanlage als auch mit anderen Partikeltherapieanlagen oder Strahlentherapieanlagen einsetzbar.

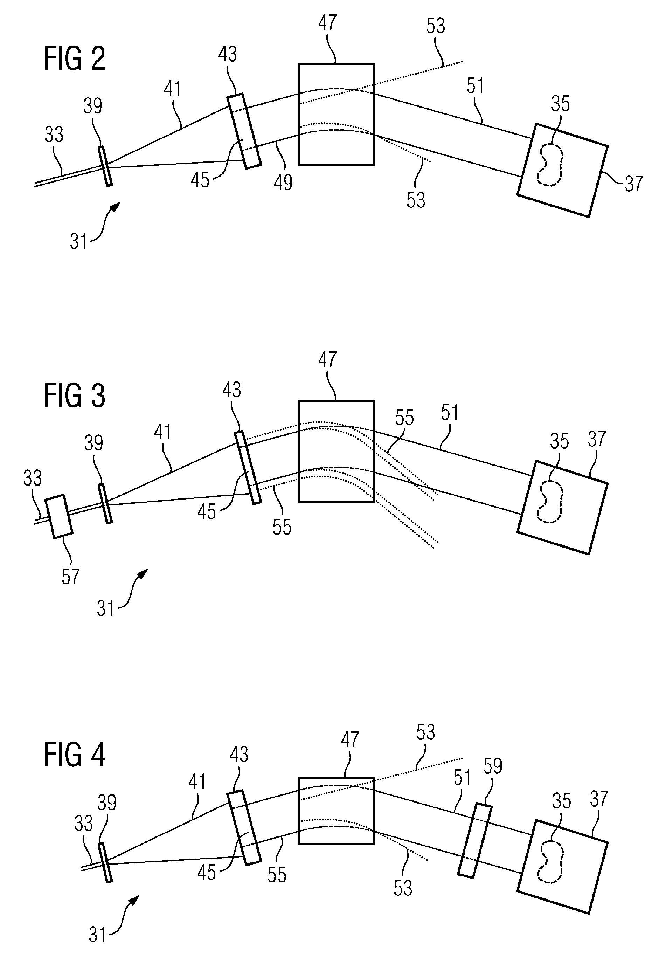

Fig. 2 zeigt schematisch den Aufbau einer Ausführungsform einer Partikelstrahlapplikationsvorrichtung 31 in einer Draufsicht. Ein in die Partikelstrahlapplikationsvorrichtung 31 eintretender Partikelstrahl 33 wird durch die Partikelstrahlapplikationsvorrichtung 31 geformt und geführt, bevor er auf ein zu bestrahlendes Zielvolumen 35, das sich innerhalb eines zu bestrahlenden Objekts 37 befindet, trifft.

Der eintretende Partikelstrahl 33 trifft auf eine Strahlaufweitungsvorrichtung 39. Die Strahlaufweitungsvorrichtung 39 erzeugt aus dem eintretenden Partikelstrahl 33, der zuvor ein im Wesentlichen punktförmiges Querschnittsprofil aufweist, einen aufgeweiteten Partikelstrahl 41 mit einem flächigen Querschnittsprofil, indem der Partikelstrahl kegelförmig aufgeweitet wird. Anschließend trifft der aufgeweitete Partikelstrahl 41 auf einen ersten Kollimator 43. Der erste Kollimator 43 weist dabei eine Öffnung 45 auf, die an das Zielvolumen 35 angepasst ist.

Nach dem der Partikelstrahl in seinem Querschnittsprofil durch den ersten Kollimator geformt ist, wird der Partikelstrahl durch ein Magnetsystem 47 geführt. Dieses Magnetsystem umfasst einen Dipolmagneten, der ein Magnetfeld erzeugt, durch das der geformte Partikelstrahl 49 in seinem Verlauf umgelenkt wird. Nachdem der Partikelstrahl umgelenkt wurde, wird der umgelenkte Partikelstrahl 51 auf das zu bestrahlende Zielvolumen 35 gerichtet.

Wenn der Partikelstrahl durch die Strahlaufweitungsvorrichtung 39 bzw. durch den ersten Kollimator 43 tritt, findet eine Wechselwirkung des Partikelstrahls mit Materie statt. Hierdurch werden im Partikelstrahl Teilchen erzeugt, die ein anderes Masse-zu-Ladungs-Verhältnis aufweisen und/oder einen anderen Impuls aufweisen als diejenigen Partikel, mit denen eine Bestrahlung erfolgen soll. Ohne Umlenkung des Partikelstrahls durch das Magnetsystem 47 würden diese Teilchen ebenfalls zumindest teilweise auf das zu bestrahlende Objekt 37 treffen. Hierdurch würde sich die Qualität der Bestrahlung des Zielvolumens 35 verschlechtern, da diese Teilchen ihre Energie an einer nicht gewünschten und auch nicht vorhersehbaren Stelle im Objekt 37 abgeben würden.

Durch das Magnetsystem 47 erfahren diese Teilchen jedoch eine andere Umlenkung als diejenigen Partikel des Partikelstrahls, mit denen die Bestrahlung erfolgen soll. In der Zeichnung ist dies durch gepunktete Kurven 53 symbolisiert. Durch das Magnetfeld werden diese Teilchen von den gewünschten Partikeln des Partikelstrahls zumindest teilweise räumlich getrennt. Auf diese Weise verbessert sich die Qualität der Bestrahlung des Zielvolumens 35.

Der erste Kollimator 43, der zur Formung des Partikelstrahls eingesetzt wird, kann dabei beispielsweise ein an das Zielvolumen 35 individuell angepasster Kollimator sein. Alternativ kann als Kollimator ein Multi-Leaf-Kollimator eingesetzt werden, dessen Öffnung flexibel an ein zu bestrahlendes Zielvolumen 35 angepasst werden kann. Eine weitere Ausführungsvariante ist ein Kollimator, der aus vorgefertigten Elementen zusammengesetzt wird, derart, dass seine Öffnung dem Zielvolumen 35 entspricht.

Fig. 3 zeigt eine weitere Ausführungsform einer Partikelstrahlapplikationsvorrichtung 31. Im Gegensatz zu der in Fig. 2 gezeigten Partikelstrahlapplikationsvorrichtung 31 ist der erste Kollimator 43' wesentlich dünner ausgebildet. Hierdurch werden die Partikel des Partikelstrahls, die nicht durch die Öffnung 45 des ersten Kollimators 43' treten, in ihrer Energie lediglich abgebremst anstatt vollständig aus dem Partikelstrahl entfernt zu werden. Durch das nachfolgende Magnetsystem 47 werden diese Partikel jedoch anders abgelenkt als diejenigen Partikel, die ungebremst durch den ersten Kollimator 43', d.h. durch die Öffnung des ersten Kollimators 43' geführt wurden. In Fig. 3 ist dies durch die gepunkteten Kurven 55 symbolisiert.

Da das Zielvolumen 35 hinter dem Magnetsystem 47 an passender Stelle positioniert ist, treffen nur diejenigen Partikel das Zielvolumen 35, die im Magnetsystem 47 eine entsprechende Ablenkung erfahren haben. Andere Partikel, wie beispielsweise diejenigen, die durch den ersten Kollimator 43' eine Abschwächung erfahren haben, werden im Magnetsystem 47 stärker abgelenkt, so dass sie nicht auf das Zielvolumen 35 treffen.

Weiterhin ist in Fig. 3 eine Tiefenmodulationsvorrichtung 57 gezeigt, die derart angeordnet ist, dass der Partikelstrahl durch die Tiefenmodulationsvorrichtung 57 tritt. Die Tiefenmodulationsvorrichtung 57 kann dabei beispielsweise ein Moderator-Wheel oder einen Ridge-Filter aufweisen. Hierdurch wird die Energie des Partikelstrahls entsprechend eingestellt, so dass der Partikelstrahl seine Energie in einer bestimmten Tiefe innerhalb des Zielvolumens 35 abgibt. Das gesamte Zielvolumen 35 kann so beispielsweise schichtweise bestrahlt werden. Hier gezeigt ist eine Anordnung der Tiefenmodulationsvorrichtung 57 im Bereich des eintretenden Partikelstrahls 33. Bei entsprechender Ausgestaltung kann die Tiefenmodulationsvorrichtung jedoch auch an anderer Stelle, beispielsweise im umgelenkten Partikelstrahl 51 oder auch vor der Umlenkung angeordnet werden.

Fig. 4 zeigt eine weitere Ausführungsform der Partikelstrahlapplikationsvorrichtung 31, bei der nach dem Magnetsystem 47 ein weiterer, zweiter Kollimator 59 angeordnet ist.

Der zweite Kollimator 59 dient dazu, das Querschnittsprofil des umgelenkten Partikelstrahls 51 erneut zu formen, bevor der Partikelstrahl auf das Zielvolumen 35 bzw. auf das Objekt 37 trifft. Hierdurch erhöht sich die Genauigkeit der Bestrahlung. Durch die Verwendung des zweiten Kollimators 59 kann der erste Kollimator 43 weniger genau an das Zielvolumen angepasst sein, da eine etwaige Feinabstimmung durch den zweiten Kollimator 59 erreicht werden kann.

Falls beispielsweise ein Multi-Leaf-Kollimator als erster Kollimator 43 eingesetzt wird, ist es nun möglich, den Multi-Leaf-Kollimator einfacher auszugestalten, da die Form des Zielvolumens weniger genau durch den Multi-Leaf-Kollimator abgebildet werden muss. Gleiches gilt für einen Kollimator, der individuell an das Zielvolumen angepasst wird, oder der aus vorgefertigten Elementen zusammengesetzt wird. Ein solcher Kollimator kann nun schneller und kostengünstiger hergestellt werden, da er weniger genau an das Zielvolumen angepasst werden muss.

Umgekehrt kann der zweite Kollimator 59 weniger genau an die Form des Zielvolumens 35 angepasst werden, wenn eine genaue Anpassung des Querschnittsprofils des Partikelstrahls bereits durch den ersten Kollimator 43 in genauer Weise erfolgt ist. Ebenso der erste Kollimator 43 kann der zweite Kollimator 59 beispielsweise ein Multi-Leaf-Kollimator, ein individuell für das zu bestrahlende Zielvolumen gefertigter Kollimator oder ein aus vorgefertigten Elementen zusammensetzbarer Kollimator sein.

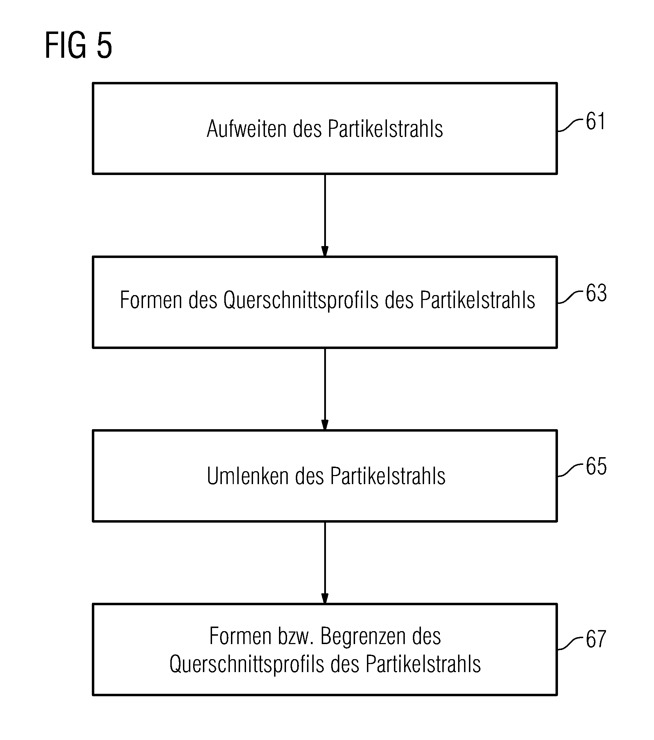

Fig. 5 zeigt einen Überblick über die Verfahrensschritte, die bei der Führung des Partikelstrahls ausgeführt werden, und die beispielsweise mit einer Ausführungsform der Partikelstrahlvorrichtung 31 gemäß Fig. 2 bis Fig. 4 ausgeführt werden können.

In einem ersten optionalen Schritt 61 erfolgt eine Aufweitung des Partikelstrahls. In einem zweiten Schritt 63 erfolgt eine Formung des Querschnittsprofils des Partikelstrahls, wie es beispielsweise anhand eines ersten Kollimators in Fig. 2 bis Fig. 4 gezeigt ist. In einem dritten Schritt 65 erfolgt eine Umlenkung des Partikelstrahls durch ein Magnetfeld, wodurch der Partikelstrahl wie beschrieben von Streustrahlung gereinigt wird. In einem vierten optionalen Schritt 67 erfolgt eine weitere Begrenzung des Querschnittsprofils des Partikelstrahls, wie es beispielsweise anhand des zweiten Kollimators in Fig. 4 gezeigt ist.

## Patentansprüche

1. Partikelstrahlapplikationsvorrichtung zum Formen und Führen eines Partikelstrahls, umfassend:
- einen ersten Kollimator mit einer Öffnung zum Formen eines Querschnittsprofils eines in den Kollimator eintretenden Partikelstrahls,
- ein Magnetsystem zur Umlenkung des Partikelstrahls, welches im Strahlverlauf des Partikelstrahls hinter dem ersten Kollimator angeordnet ist, wobei mit dem Magnetsystem ein Magnetfeld erzeugbar ist, mit dem der Partikelstrahl spektral auffächerbar ist.

2. Partikelstrahlapplikationsvorrichtung nach Anspruch 1, wobei die Öffnung des ersten Kollimators einem zu bestrahlenden Zielvolumen angepasst ist.

3. Partikelstrahlapplikationsvorrichtung nach Anspruch 1 oder 2, wobei
der erste Kollimator zum Formen des Querschnittsprofils des in den ersten Kollimator eintretenden Partikelstrahls derart ausgebildet ist, dass durch den Kollimator die Energie von Partikeln des Partikelstrahls, die nicht durch die Öffnung des Kollimators treten, lediglich abschwächbar ist.

4. Partikelstrahlapplikationsvorrichtung nach einem der Ansprüche 1 bis 3, wobei
die durch das Magnetsystem erzeugte Umlenkung des Partikelstrahls mehr als 5°, insbesondere mehr als 10° beträgt.

5. Partikelstrahlapplikationsvorrichtung nach einem der Ansprüche 1 bis 4, wobei
das Magnetsystem zumindest einen Dipolmagneten umfasst.

6. Partikelstrahlapplikationsvorrichtung nach einem der Ansprüche 2 bis 5, wobei
der erste Kollimator ein Kollimator aus einer Gruppe ist, welche einen individuell für das zu bestrahlende Zielvolumen gefertigten Kollimator, einen aus vorgefertigten Elementen zusammensetzbaren Kollimator und einen Multi-Leaf-Kollimator umfasst.

7. Partikelstrahlapplikationsvorrichtung nach einem der Ansprüche 1 bis 6, zusätzlich umfassend:
- eine Strahlaufweitungsvorrichtung, die im Strahlverlauf vor dem ersten Kollimator angeordnet ist.

8. Partikelstrahlapplikationsvorrichtung nach einem der Ansprüche 1 bis 7, zusätzlich umfassend:
- einen im Strahlverlauf hinter dem Magnetsystem angeordneten zweiten Kollimator zum Begrenzen des Querschnittsprofils des aus dem Magnetsystem austretenden Partikelstrahls.

9. Partikelstrahlapplikationsvorrichtung nach Anspruch 8, wobei
der zweite Kollimator ein Kollimator aus einer Gruppe ist, welche einen individuell für das zu bestrahlende Zielvolumen gefertigten Kollimator, einen aus vorgefertigten Elementen zusammensetzbaren Kollimator oder einen Multi-Leaf-Kollimator umfasst.

10. Partikelstrahlapplikationsvorrichtung nach einem der Ansprüche 1 bis 9, zusätzlich umfassend:
- eine Tiefenmodulationsvorrichtung, die im Strahlverlauf des Partikelstrahls anordenbar ist, zur Energievariation des durch die Tiefenmodulationsvorrichtung tretenden Partikelstrahls.

11. Bestrahlungsvorrichtung, umfassend:
- zumindest eine Partikelstrahlapplikationsvorrichtung nach einem der Ansprüche 1 bis 10,
- zumindest eine Quelle zur Erzeugung von Partikeln, und
- zumindest eine vor der Partikelstrahlapplikationsvorrichtung angeordnete Beschleunigungsvorrichtung zur Beschleunigung der Partikel und zur Erzeugung des Partikelstrahls aus den beschleunigten Partikeln.

12. Verfahren zur Führung eines Partikelstrahls, aufweisend folgende Verfahrensschritte:
- Formen eines Querschnittsprofils eines Partikelstrahls,
- Führen des geformten Partikelstrahls durch ein Magnetfeld, wodurch der Partikelstrahl umgelenkt und spektral aufgefächert wird.

13. Verfahren nach Anspruch 12, wobei
dass Querschnittsprofil des Partikelstrahls derart geformt wird, dass der Partikelstrahl an ein zu bestrahlendes Zielvolumen angepasst ist.

14. Verfahren nach Anspruch 12 oder 13, wobei bei dem Formen des Querschnittsprofils des Partikelstrahls der Partikelstrahl durch einen Kollimator mit einer Öffnung geführt wird, wobei bei dem Durchtritt des Partikelstrahls durch den Kollimator diejenigen Partikel, die nicht durch die Öffnung des Kollimators treten, in ihrer Energie lediglich abgeschwächt werden.

15. Verfahren nach einem der Ansprüche 12 bis 14, wobei bei dem Führen des geformten Partikelstrahls durch das Magnetfeld der Partikelstrahl um mehr als 5°, insbesondere mehr als 10° umgelenkt wird.

16. Verfahren nach einem der Ansprüche 12 bis 15, wobei der Partikelstrahl vor dem Formen des Querschnittsprofils des Partikelstrahls aufgeweitet wird.

17. Verfahren nach einem der Ansprüche 12 bis 16, wobei nach dem Führen durch das Magnetfeld das Querschnittsprofil des Partikelstrahls begrenzt wird.

18. Verfahren nach einem der Ansprüche 12 bis 17, wobei im Strahlverlauf durch eine Tiefenmodulationsvorrichtung die Energie der durch die Tiefenmodulationsvorrichtung tretenden Partikel des Partikelstrahls abgeschwächt wird.
